(19) Europäisches Patentamt
European Patent Office
Office européen des brevets

(11) Numéro de publication: **0 112 743**
**B1**

(12) # FASCICULE DE BREVET EUROPEEN

(45) Date de publication du fascicule du brevet: **11.03.87**

(51) Int. Cl.[4]: **C 07 F 9/65**, A 61 K 31/675

(21) Numéro de dépôt: **83402256.8**

(22) Date de dépôt: **23.11.83**

(54) **Nouveaux dérivés de tricyclophosphazènes, procédé pour leur préparation et leur application en tant que médicament.**

(30) Priorité: **25.11.82 FR 8219768**

(43) Date de publication de la demande:
**04.07.84 Bulletin 84/27**

(45) Mention de la délivrance du brevet:
**11.03.87 Bulletin 87/11**

(84) Etats contractants désignés:
**AT BE CH DE FR GB IT LI LU NL SE**

(56) Documents cités:
**EP - A - 0 022 407**
**FR - A - 2 104 915**
**NL - A - 8 007 107**

**EUROPEAN JOURNAL OF CANCER, vol. 15, no. 5, 1979 (GB), J.F. LABARRE et al.: "Antitumor activity of some cyclophosphazenes", pages 637-643.**
**RECUEIL, JOURNAL OF THE ROYAL NETHERLANDS CHEMICAL SOCIETY, vol. 101, juillet-août 1982, A.A. SMAARDIJK et al.: "A simple synthesis of glycino ethyl ester derivatives of hexachlorocyclotriphosphazene", pages 270-271.**
**JOURNAL OF THE CHEMICAL SOCIETY, DALTON TRANSACTIONS Inorganic Chemistry, vol. 5, 1980, S.S. KRISHNAMURTHY et al.: "Studies of Phosphazenes. Part 10. Spirocyclic derivatives of cyclotriphosphazatrienes", pages 840-844.**
**CHEMICAL ABSTRACTS, vol. 99, no. 7, 15 août 1983, page 17, réf. no. 47548u, Columbus Ohio (US)**

(73) Titulaire: **CENTRE NATIONAL DE LA RECHERCHE SCIENTIFIQUE (CNRS), 13 Quai Anatole France, F-75700 Paris (FR)**

(72) Inventeur: **Labarre, Jean-François, 30 rue des Géraniums, F-31400 Toulouse (FR)**
Inventeur: **Guerch, Guy, Le Fournier Regibue, F-31320 Castanet (FR)**
Inventeur: **Levy, Gaston, 2 rue St. Amans, F-31130 Balma (FR)**
Inventeur: **Sournies, François, 5 rue du Moulin, F-31520 Ramonville St. Agne (FR)**

(74) Mandataire: **Warcoin, Jacques et al, Cabinet Régimbeau 26, avenue Kléber, F-75116 Paris (FR)**

**EP 0 112 743 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés chimiques nouveaux, des procédés pour leur préparation et l'application de ces composés à titre de médicaments.

C'est en 1979 (J-F. LABARRE, J-P. FAUCHER, G. LEVY, F. SOURNIES, S. CROS et G. FRANCOIS, Europ. J. Cancer, *15,* (1979) 637) que l'activité antitumorale in vivo sur les leucémies P388 et L1210 et sur le mélanome B16 sous-cutané (greffés chez des souris DBA/2 femelles) de l'hexaziridinocyclotriphosphazène $N_3P_3Az_6$ fut chiffrée pour la première fois.

Les laboratoires de l'EORTC apportèrent ensuite les preuves que cette activité s'exerce également d'une manière significative sur 18 autres tumeurs parmi lesquelles on peut citer le carcinome du colon de la ligne 26, le carcinome du poumon de Lewis, le mastocytome P815, le carcinome mammaire de la ligne 16, l'épendymoblastome et le sarcome de Yoshida.

Ce spectre d'activité très étendu sur les tumeurs murines joint à la faible mutagénèse de cette molécule (nom de code EORTC: MYKO 63) en faisaient une droque dont le développement en vue d'une utilisation en clinique humaine pouvait être raisonnablement envisagée.

Il s'avéra toutefois rapidement que le MYKO 63 était en réalité pénalisé par l'apparition d'une toxicité cumulative facheuse lors de protocoles de polyinjections tu type 5 Q3D ou 5 Q4D. Le MYKO 63 était donc une substance antitumorale trop lente quant à sa cinétique d'action sur la tumeur et à sa vitesse d'excrétion.

Afin d'écarter les inconvénients liés à l'utilisation du MYKO 63, il a été mis au point des dérivés présentant des structures voisines, les cyclodiphosphathiazènes $(NPAz_2)2NSOX$ (X = F : SOF ; X = Ph : SOPhi ; X = Az : SOAz).

Le SOF, le SOPhi et surtout le SOAz présentent une activité remarquable, sensiblement égale en monoinjection à celle du MYKO 63, mais avec disparition de toute toxicité cumulative même lors de protocoles Q3D et Q4D lourds.

Le SOAz est actuellement en phase 2 clinique humaine.

En dépit du succès ainsi enregistré, en particulier avec le SOAz, quant à l'amélioration de la pharmacocinétique du MYKO 63 par substitution d'un atome de phosphore de son cycle par un atome de soufre, il n'en demeure pas moins que les doses nécessaires à l'activité thérapeutique du SOAz restent fortes (du domaine de l'allopathie) et qu'une proportion non négligeable ~ 40%) de la quantité injectée est retrouvé intacte dans les urines des patients au cours des 12 heures qui suivent l'injection. Ceci prouve à l'évidence que le SOAz injecté n'atteint pas dans sa totalité la tumeur, une part trop importante du médicament ne remplissant donc pas son rôle.

La présente invention a donc pour objet de nouveaux composés ayant un meilleur pouvoir de pénétration dans la cellule maligne afin d'accroître la quantité de matière active atteignant la tumeur.

Il s'agit de composé de formule I:

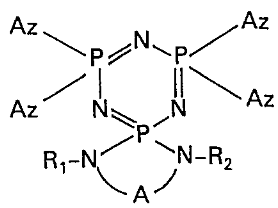

(I)

dans laquelle:

Az est un radical 1-aziridinyle non substitué ou substitué par 1 à 4 radicaux alkyles ou alkoxy en $C_1$ à $C_3$ ;

A est une chaîne alcoylène, alcénylène ou alcynylène, droite ou ramifiée, substituée ou non, qui peut être interrompue par un ou plusieurs radicaux divalents $-NR_3-$, -S- ou -O-, la chaîne -A- pouvant être substituée par un ou plusieurs atomes d'halogène, radicaux imino, amino, aryle non substitué ou substitué par un ou plusieurs atomes d'halogène ou radicaux alkyles en $C_1$ à $C_3$;

$R_1$, $R_2$ et $R_3$ peuvent représenter, indépendamment l'un de l'autre:

un atome d'hydrogène,
un radical $C_1$-$C_7$-alkyle,
un radical $C_1$-$C_7$-alcényle,
un radical $C_1$-$C_7$-alcynyle,
un radical monocyclique aryle,
les radicaux $R_1$, $R_2$ et $R_3$ peuvent être substitués par un atome d'halogène, les radicaux imino, amino, aryle non substitué ou substitué par un ou plusieurs atomes d'halogène ou radicaux alkyles en $C_1$ à $C_3$.

Dans les composés de formule I le radical Az est, de préference, un radical 1-aziridinyle non substitué.

La chaîne -A- peut comprendre de 1 à 5 atomes de carbone.

-A- est de préférence une chaîne alcoylène. La chaîne -A- peut être une chaîne carbonée non interrompue ou bien interrompue par un ou plusieurs radicaux $-NR_3-$, -0- ou -S-, de préférence par 1 à 4 radicaux de ce type.

De façon préférentielle, les radicaux $R_1$, $R_2$ et $R_3$ sont :

- un atome d'hydrogène,
- un radical alkyle, non substitué ou substitué.

Par «radical alkyle», il faut entendre plus particulièrement les radicaux alkyles inférieurs, droits ou ramifiés, ayant en particulier de 1 à 7 atomes de carbone comme les radicaux méthyle, éthyle, n-propyle et isobutyle.

Par le terme «radical alcényle», il faut entendre plus particulièrement les radicaux alcényles inférieurs, droits ou ramifiés, ayant en particulier de 2 à 7 atomes de carbone, comportant une ou plusieurs insaturations éthyléniques, comme le radical éthylényle par exemple.

Par «radical alcynyle», il faut entendre plus particulièrement les radicaux alcynyles inférieurs, droits ou ramifiés, ayant en particulier de 2 à 7 atomes de carbone, comme les radicaux acétynyle ou propargyle.

Par «radical aryle», il faut entendre essentiellement des radicaux monocycliques, en particulier le radical phényle, le radical aryle pouvant être substitué de différentes façons, en particulier par des radicaux alkyles, comme dans les radicaux tolyle ou xylyle par exemple.

Parmi les substituants sur les carbones de la chaîne -A-, il faut citer notamment les radicaux imino ou amino.

De même, pour les substituants $R_3$, il faut citer notamment les radicaux alkylamino.

La présente invention concerne en particulier les composés de formule I':

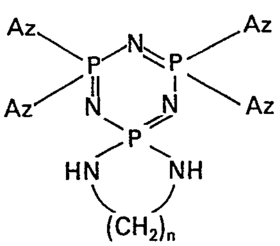

(I')

n étant un nombre entier de 1 à 5 et de préférence un nombre choisi parmi 2, 3, 4 et 5.

Lorsque n = 3, le composé est de formule :

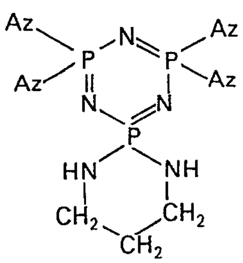

Ce produit sera dénommé SPIRODIAM 3.

Lorsque n = 4, le composé est de formule:

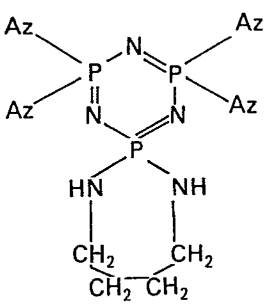

Ce produit sera dénommé SPIRODIAM 4.

La présente invention concerne également des procédés de préparation des composés de formule I dans lesquels:

a) on fait réagir la polyamine de formule II:

$$H(R_1N)—A—(NR_2)H \qquad (II)$$

sur le composé gem dichloré de formule III:

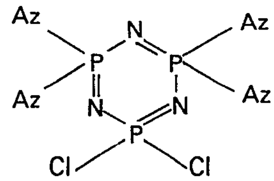

(III)

ou bien,

b) on fait réagir l'aziridine non substituée ou substituée en excès sur le composé de formule IV:

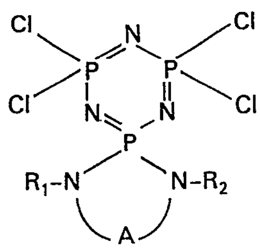

(IV)

Le composé de formule III sera parfois appelé MYCLAz.

Les composés de formule III et IV peuvent être préparés à partir du dérivé hexachloré $N_3P_3Cl_6$, soit en faisant réagir l'aziridine pour préparer le composé de formule III, soit en faisant réagir la polyamine $H(R_1N)—A—(NR_2)H$ pour obtenir le composé de formule IV.

Ces différentes réactions sont effectuées de préférence en présence d'un agent fixateur de HCl qui est, de préférence, une amine ne réagissant pas sur le noyau de base telle qu'une amine tertiaire trialcoylamine par exemple, soit en présence du réactif en excès par rapport à la réaction que l'on désire privilégier.

Ainsi, dans la deuxième étape du procédé, pour passer de III ou IV à I on peut utiliser un large excès du réactif supérieur à 2:1 si nécessaire pour III, ou 4:1 si nécessaire pour IV, mais en présence d'un accepteur d'acide on pourra utiliser 1:1 pour III et 2:1 pour IV.

Ces réactions sont conduites de préférence dans un solvant tel qu'un éther ou un solvant halogéné ou un mélange de tels solvants, par exemple un mélange d'éther de pétrole 60-80° et de dichlorométhane dans le rapport 4:1 et se déroulent de préférence à une température comprise entre -20 et +20°C, par exemple à une température voisine de 0°C et en atmosphère inerte ($N_2$ ou Ar soigneusement desséché).

Parmi les polyamines utilisables dans la préparation des composés selon l'invention, il faut citer:

- la propylène-diamine, la putrescine et la cadavérine qui conduisent à des composés I'.

Enfin, le produit de la réaction de formule I peut être purifié par des procédés connus tels que l'extraction par solvant, la cristallisation, la chromatographie en phase liquide, la chromatographie en phase liquide à haute performance («HPCL») par exemple.

Les différents produits de départ utiles dans la mise en œuvre des procédés selon la présente invention peuvent être préparés, notamment, par les méthodes décrites dans:

- Y. KOBAYASHI, L.A. CHASIN et L.B. CLAPP, Inorg. Chem. *2*, 212 (1963),
- R. RATZ, E. KOBER, C. GRUNDMANN et G. OTTMANN, Inorg. Chem., *3*, 757 (1964) et brevet des E.U.A. n° 3197464, 27 juillet 1965,
- G.GUERCH, J-F. LABARRE, F. SOURNIES, M. MANFAIT, F. SPREAFICO et S. FILIPPESCHI, Inorg. Chim. Acta, *66*, 175-183 (1982), pour le gem-$N_3P_3Az_4Cl_2$, et
- M. BECKE-GOEHRING et B. BOPPEL, Z. Anorg. Allg. Chem., *322*, 239 (1963).
- S.S. KRISHNAMURTHY, K. RAMACHANDRAN, A.R. VASUDEVA MURTHY, R.A. SHAW et M. WOODS, Inorg. Nucl. Chem. Lett., *13*, 407 (1977),
- G. GUERCH, M. GRAFFEUIL, J-F. LABARRE, R. ENJALBERT, R. LAHANA et F. SOURNIES, J. Mol. Struct., *85*, 000 (1982),
- G. GUERCH, J-F. LABARRE, R. ROQUES et F. SOURNIES, J. Mol. Struct., *85*, 000 (1982) pour les spiro-$N_3P_3Cl_4[POLYAM_{m,n}]$.

Enfin, la présente invention concerne, à titre de médicament nouveau, certains composés tels que décrits précédemment ainsi que des compositions pharmaceutiques comprenant à titre de principe actif au moins l'un de ces composés et, en particulier, des compositions pharmaceutiques administrables par voie parentérale.

Parmi les compositions administrables par voie parentérale il faut citer les compositions injectables, en particulier par voie intrapéritonéale et intraveineuse, bien que l'on puisse utiliser d'autres compositions telles que les pellets. Les produits en cause étant en général assez solubles dans l'eau, on utilisera de préférence comme support pharmaceutique un solvant aqueux, par exemple une solution physiologique de chlorure de sodium. Bien entendu, dans le cas de produits difficiles à solubiliser on peut utiliser des tampons fixant le pH ou éventuellement un solvant non aqueux tel que des esters, des aclools, des polyols ou diverses huiles en combinaison avec des agents émulsifiants, on peut également utiliser des suspensions, en particulier des hydroxypropylcelluloses.

Bien que les compositions pharmaceutiques selon la présente invention soient de préférence des compositions injectables, on peut également utiliser des compositions administrables par voie digestive, voie sublinguale, orale ou rectale, ces compositions pouvant se présenter sous forme solide, comprimés, cachets, granulés, gélules, suppositoires par exemple, ou sous forme liquide, gouttes et ampoules.

Dans le cas des compositions par voie orale, celles-ci sont formulées avec des supports connus solides tels que gélatine, gomme arabique, lactose, amidon, polyalkylène glycol, carboxyméthylcellulose par exemple.

Dans le cas de suppositoires, on peut utiliser des polyéthylène glycol, de la lanoline à titre de véhicule.

Les compositions selon la présente invention peuvent également être applicables par voie topique, en particulier sous forme de pommade ou de gel applicable sur la peau avec un véhicule inerte tel que la vaseline, les polyéthylène glycol ou d'autres excipients gras; il est possible également de prévoir un agent facilitant la pénétration cutanée du principe actif.

De façon générale, les compositions selon la présente invention peuvent contenir des adjuvants variés tels que conservateurs, stabilisants, mouillants, émulsifiants, agents de texture, de delitement, aromatisation, colorants. Les composés selon la présente invention se sont révélés particulièrement actifs dans le traitement de certaines tumeurs. Ils se sont en particulier révélés très actifs sur les deux tumeurs standard que sont la leucémie P388 et le mastocytome P815.

Ces composés sont utilisables essentiellement par injection, en particulier par voie intra-péritonéale ou par voie intra-veineuse. Ces composés étant très solubles dans l'eau, la réalisation de compositions injectables ne présente aucun problème. Les doses appliquées sont, bien entendu, fonction, dans chacun des cas, du type de tumeur à traiter ainsi que de l'état général du malade, et peuvent varier dans une très large mesure, en particulier entre 1 mg/kg/jour et 100 mg/kg/jour, en une ou plusieurs injections dont la fréquence peut être variable.

Les exemples suivants sont destinés à illustrer la préparation et les activités de certains des composés selon la présente invention sans pour autant la limiter.

Exemple 1

Préparation du composé spiro-$N_3P_3Az_4$ $[HN-(CH_2)_3-NH]$ en passant par la synthèse du composé gem-$N_3P_3Az_4Cl_2$

Une solution de 87,0 mmoles de propylènediamine $[H_2N-(CH_2)_3-NH_2]$ fraîchement distillée dans 50 ml d'un mélange (4:1) d'éther de pétrole 60-80 °C et de $CH_2Cl_2$ (appelé ci-dessous S) est ajoutée goutte à goutte pendant 2 heures à une solution de 32,0 mmoles de gem-$N_3P_3Az_4Cl_2$ dans 150 ml du même solvant S sous agitation vigoureuse et à température ordinaire. L'agitation magnétique est maintenue durant toute une nuit. Le chlorhydrate de propylènediamine formé est éliminé par filtration et le solvant de la solution mère est évaporé sous vide. Le produit de réaction brut donne deux taches en chromatographie sur couche mince (avec le méthanol comme éluant) à Rf : 0,17 et 0,68.

La recristallisation de ce produit brut dans 20 volumes de $CCl_4$ anhydre par évaporation partielle du solvant et refroidissement subséquent de la solution conduit à un composé cristalline (Rf = 0,68) présentant un point de fusion de 134°C, le rendement étant de 80%.

Le composé présente les caractéristiques analytiques suivantes :

| Analyse | | | | |
|---|---|---|---|---|
| calculé: | C 35,20 | H 6,44 | N 33,59 | P 24,76 |
| trouvé: | C 34,98 | H 6,40 | N 33,38 | P 24,59 |
| | 35,10 | 6,50 | 33,70 | 24,83 |

Spectre de masse

m/z 375 $(M)^+$ 38,2%, 333 $(M\text{-}1Az)^+$ 100%, 292 $(M\text{-}2Az)^+$ 29,2%,
249 $(M\text{-}3Az)^+$ 17,9%, 208 $(M\text{-}4Az)^+$ 12,9%,
304 $(N_3P_33Az_4)^+$ 9,5%, 261 $(N_3P_3Az_3)^+$ 10,6%,
220 $(N_3P_3Az_2)^+$ 5,0%, 177 $(N_3P_3Az_3)^+$ 3,3%,
135 $(N_3P_3)^+$ 1,1%.

$^{31}p$ RMN en solution dans $CH_2Cl_2$ (par rapport à $H_3PO_4$ 85%)

Spectre $AX_2$ comportant un doublet à 38,34 et 37,45 ppm et un triplet à 19,69, 18,80 et 17,91 ppm dont les intensités sont dans le rapport 2:1. J(P-P) = 32,36 Hz.

IR ($cm^{-1}$) (KBr)

3360 w, 3255 m, 3050 w, 2985 m, 2958 w, 2925 vw, 2880 w, 2860 w, 1255 s, 1180 vs, 1160 m, 1150 m, 1080 s, 930 vs, 870 m, 840 m, 800 m, 703 s, 640 s, 510 s, 497 sh, 440 m.

Structure aux rayons X

Groupe d'espace $P2_1/a$, a = 18,369(3)Å, b = 9,715(1)Å, c = 9,722(2)Å, α 90°, β = 94,79(1)°, γ = 90°, V = 1728,9(5)Å$^3$, Z = 4 (voir figures 1 et 2), R = 0,047.

Cette structure met en évidence la non planéité de la boucle spiro, le cycle $N_3P_3$ étant pour sa part rigoureusement plan aux erreurs d'expérience près.

La figure 3 montre, en outre, les modifications conformationnelles des 4 pales aziridinyles induites par la greffe de la boucle -[HN-$(CH_2)_3$-NH]- sur le phosphore portant 2Cl du composé $N_3P_3Az_4Cl_2$.

Exemple 2

Préparation du composé spiro-$N_3P_3Az_4$ [HN-$(CH_2)_3$-NH] en passant par la synthèse du composé spiro-$N_3P_3Cl_4$[HN-$(CH_2)_3$-NH]

Une solution de 48,5 mmoles d'aziridine fraîchement distillée dans 35 ml du solvant S est ajoutée goutte à goutte en 1 heures à une solution de 11,0 mmoles de spiro-$N_3P_3Cl_4$ [HN-$(CH_2)_3$-NH] et de 44,5 mmoles de $Et_3N$ dans 120 ml de solvant S. La réaction est conduite à 0°C sous agitation vigoureuse et en atmosphère inerte ($N_2$ ou Ar sec). On laisse le mélange réactionnel revenir à température ambiante puis on agite encore pendant 24 heures à cette même température.

Le chlorhydrate de triéthylamine est alors filtré et le solvant de la solution mère évaporé sous vide. Le produit de réaction brut donne ici encore 2 taches en chromatographie sur couche mince à Rf = 0,17 et 0,68.

La recristallisation dans 20 volumes de $CCl_4$ par évaporation partielle du solvant et refroidissment de la solution conduit au dérivé cristallisé attendu. Point de fusion : 134°C, rendement : 87%.

Ce composé présente des caractéristiques analytiques identiques à celles décrites ci-dessus pour l'échantillon obtenu par la première méthode.

Exemples 3 et 4

Préparations du composé spiro-$N_3P_3Az_4$ [HN-$(CH_2)_4$-NH]

La synthèse de ce composé peut être réalisée en suivant l'un ou l'autre des deux modes opératoires décrits ci-dessus dans les exemples 1 et 2 à propos du spiro-[HN-$(CH_2)_3$-NH] et en remplaçant, dans le procédé de l'exemple 1, la propylènediamine par la putrecine, et dans le procédé de l'exemple 2, le spiro-$N_3P_3Cl_4$-[NH-$(CH_2)_3$-NH] par le spiro-$N_3P_3Cl_4$[NH-$(CH_2)_4$-NH].

Quelle que soit la méthode utilisée, le rendement en produit pur est supérieur ou égal à 90%.

Les caractéristiques analytiques de ce composé sont les suivantes:

Chromatographie sur couche mince

Rf = 0,41 avec $CH_3OH$ comme éluant.

| Analyse | | | | |
|---|---|---|---|---|
| calculé: | C 37,02 | H 6,73 | N 32,38 | P 23,87 |
| trouvé: | C 36,93 | H 6,70 | N 32,08 | P 23,75 |
| | 37,10 | 6,75 | 32,47 | 23,78 |

Point de fusion (non corrigé): 171°C

Spectre de masse

m/z 389 $(M)^+$ 83,3%, 347 $(M\text{-}1Az)^+$
96,8%, 304 $(M\text{-}2Az)^+$ 100%,
263 $(M\text{-}3Az)^+$ 30,9%, 220 $(M\text{-}4Az)^+$ 20,6%,
261 $(N_3P_3Az_3)^+$ 36,5%, 177 $(N_3P_3Az)^+$ 17,4%,
135 $(N_3P_3)^+$ 3,9%.

On peut remarquer que les pics à m/z 304 et 220 peuvent être aussi attribués aux fragments $(N_3P_3Az_4)^+$ et $(N_3P_3Az_2)^+$ en raison de l'identité des masses de 2Az et du fragment [HN-$(CH_2)_4$-NH], lesquelles sont toutes deux égales à 84.

$^{31}p$ RMN en solution dans $CH_2Cl_2$ (par rapport à $H_3PO_4$ 85%)

Spectre $AX_2$ comportant un doublet à 38,74 et 37,77 ppm et un triplet à 23,65, 22,68 et 21,71 ppm dont les intensités sont dans le rapport 2:1. J(P-P) = 35,29 Hz.

IR ($cm^{-1}$) (KBr)

3270 m, 3060 w, 2990 m, 2920 m, 2880 vw, 2865 vw, 1260 s, 1230 s, 1190 vs, 1170 s, 1120 s, 1080 w, 1055 m, 955 m, 925 vw, 855 m, 845 m, 810 m, 720 w, 705 m, 690 m, 640 s, 525 w, 435 w.

Les composés de départ mis en œuvre dans les exemples précédents peuvent être préparés comme cela est décrit dans les documents suivants ou bien par des procédés analogues.

– gem-$N_3P_3Az_4Cl_2$:

Y. KOBAYASHI, L.A. CHASIN et L.B. CLAPP, Inorg. Chem., *2,* 212 (1963),

R. RATZ, E. KOBER, C. GRUNDMANN et G. OTTMANN, Inorg. Chem., *3,* 757 (1964) et brevet des E.U.A. n° 3 197 646, 27 juillet 1965

G. GUERCH, J-F. LABARRE, F. SOURNIES, M. MANFAIT, F. SPREAFICO et S. FILIPPESCHI, Inorg. Chim. Acta, *66,* 175–183 (1982);

– spiro-$N_3P_3Cl_4$[HN-$(CH_2)_3$-NH]

G. GUERCH, M. GRAFFEUIL, R. ENJALBERT, R. LAHANA, J-F. LABARRE et F. SOURNIES, J. Mol. Struct., *85,* 000 (1982):

– spiro-$N_3P_3Cl_4$[HN-$(CH_2)_4$-NH]

G. GUERCH, J-F. LABARRE, R. ROQUES et F. SOURNIES, J. Mol. Struct., *85,* 000 (1982).

Propriétés pharmacologiques

Les composés mentionnés précédemment ont été testés sur le plan de leur activité pharmacologique.

On a tout d'abord observé que la solubilité des deux composés des exemples 1 à 4 est égale ou supérieure à 20 g/l ce qui est donc largement suffisant pour la réalisation de formes injectables en solution aqueuse.

La toxicité de ces composés a été déterminée sur des souris femelles Swiss ou CD2F1.

La léthalité qui apparaît systématiquement aux jours 5 et 6 après l'administration a été enregistrée en fonction de la dose inoculée et a permis de déduire la $DL_0$ qui correspond à la dose maximale non léthale. Les résultats ne dépendent pas de l'espèce de souris utilisée. On a constaté que:

– la $DL_0$ du composé spiro-$N_3P_3Az_4$ [HN-$(CH_2)_3$-NH] (ci-après SPIRODIAM 3) est de 75 mg/kg,

– la $DL_0$ du composé spiro-$N_3P_3Az_4$ [HN-$(CH_2)_4$-NH] (ci-après SPIRODIAM 4) est de 125 mg/kg.

Activité antitumorale

Les essais d'activité antitumorale ont été conduits par le Screening and Pharmacology Group de l'EORTC implanté à l'Institut Mario Negri à Milan.

Ces essais ont été conduits soit en monoinjection à différentes doses aux jours J+1 suivant la greffe de la tumeur, soit en polyinjections chroniques Q1D sur des souris CD2F1 femelles.

Les résultats observés sont rassemblés dans le tableau 1 ci-après.

Dans ce tableau:

$$ILS\% = \frac{T-C}{C} \times 100$$

T (en jours) étant la durée moyenne de vie des souris traitées C (en jours) étant la durée moyenne de vie des souris témoins. QnD signifie une injection tous les n jours à partir du jour J+1, ainsi «5 injections Q1D» signifie 1 injection quotidienne de J+1 à J+5.

Le tableau 1 appelle les remarques essentielles suivantes:

1. Le SPIRODIAM 3 et le SPIRODIAM 4 guérissent en polyinjections Q1D aussi bien la leucémie P388 que le mastocytome P815. La guérison est également atteinte en monoinjection (à 100 mg/kg, soit au 4/5 de la $DL_0$) avec le SPIRODIAM 4 dans le cas du P815.

2. Compte tenu des valeurs de $DL_0$ indiquées plus haut, il s'avère que les indices thérapeutiques (définis comme le rapport de la $DL_0$ à la dose minimale conduisant à un ILS significatif, c'est-à-dire à un ILS de 25%) des deux composés sont très largement supérieurs à 10. Des indices aussi élevés sont uniques en chimiothérapie du cancer. Cela signifie que l'activité antitumorale se manifeste déjà en monoinjection au 1/10 de la $DL_0$ et qu'une polyinjection chronique Q1D répétée n fois de cette dose très faible permet d'atteindre la guérison en minimisant les éventuels effets secondaires de la drogue.

3. La pharmacocinétique d'action sur la tumeur et de l'excrétion du médicament semblement particulièrement favorables: 9 injections Q1D à 20 mg/kg de SPIRODIAM 3 ou de SPIRODIAM 4 (dans le cas du P815) correspondent en effet à une dose totale sur 9 jours de 180 mg, soit respectivement 2,4 et 1,5 fois la $DL_0$ de ces drogues. Or, ces deux protocoles n'induisent aucune toxicité cumulative.

Le même effet est observé dans le cas de la P388 où 5 injections Q1D à 40 mg/kg, correspondant respectivement à 2,7 et 1,6 fois la $DL_0$, n'induisent elles non plus de toxicité cumulative.

4. Les figures 4 à 9 traduisent les relations ILS = f(dose) pour le SPIRODIAM 3 et le SPIRODIAM 4 en monoinjection et en polyinjections Q1D sur la P388 et le P815. Ces figures permettent les commentaires suivants:

a) SPIRODIAM 3 et SPIRODIAM 4 en monoinjection sur la P388 (fig. 4–5) se caractérisent par une relation ILS = f (dose) linéaire,

b) SPIRODIAM 3 et SPIRODIAM 4 en monoinjection sur le P815 (fig. 6–7) se caractérisent par une relations ILS = f (dose) à tendance exponentielle analogue à celle obtenue avec le SOAz dans le cas de la P388 avec des souris DBA/2 femelles,

c) SPIRODIAM 3 et SPIRODIAM 4 en polyinjections Q1D sur la P388 et le P815 (Fig. 8–9) se caractérisent par des relations ILS = f (dose) sensiblement linéaires.

5. Les résultats suivants on été observés:

a) Pour le carcinome prostatique:

9 injections Q1D à 30 mg/kg
T/C (%) SPIRODIAM 3: 158%
SPIRODIAM 4: 154%,

b) Pour le carcinome du rhin
9 injections Q1D à 20 mg/kg de SPIRODIAM 3
T/C (%): 223%,

9 injections QID à 10 mg/kg de SPIRODIAM4
T/C (%): 155%,

c) Pour le carcinome du colon de la ligne 26:
9 injections Q1D à 10 mg/kg de SPIRODIAM3
T/C (%): 132%
9 injections Q1D à 20 mg/kg SPIRODIAM4
T/C (%): 132%,

d) Pour le carcinome des poumons de Lewis;
9 injections Q1D à 10 mg/kg de SPIRODIAM3
TWI %: 43,7

Le SPIRODIAM4 n'est actif qu'au voisinage de la dose toxique.

En conclusion, le SPIRODIAM3 et le SPIRODIAM4 constituent des améliorations indiscutables du MYKO 63, du MYCLAz et du SOAz à la fois du point de vue de l'indice thérapeutique et de l'activité antitumorale sur les tumeurs murines.

Ce dernier point est clairement illustré par le tableau 2 dans lequel sont rapprochés les ILS % obtenus avec le MYKO 63, le MYCLAz, le SOAz, le SPIRODIAM 3 et le SPIRODIAM 4 sur la P388 et le P815 lors de la mono-injection d'une même fraction de la $DL_0$.

Tableau I

| Tumeur | Protocole (par voie i.p.) Compose | Dose (mg/kg/ jour) | ILS% |
|---|---|---|---|
| P388 | 1 injection J+1 | 5 | 30 |
| | Spirodiam 3 | 12,5 | 50 |
| | | 50 | 100 |
| | | 100 | (94)* |
| | 5 injections Q1D | 2,5 | 45 |
| | | 10 | 81 |
| | | 20 | 150 |
| | | 40 | 204 |
| | 1 injection J+1 | 12,5 | 50 |
| | Spirodiam 4 | 50 | 74 |
| | | 100 | 103 |
| | 5 injection Q1D | 2,5 | 50 |
| | | 10 | 90 |
| | | 20 | 155 |
| | | 40 | 210 |
| P815 | 1 injection J+1 | 12,5 | 55 |
| | Spirodiam 3 | 25 | 70 |
| | | 50 | 150 |
| | | 100 | (95)* |
| | 9 injections Q1D | 1,25 | 50 |
| | | 2,5 | 55 |
| | | 5 | 105 |
| | | 10 | 155 |
| | | 20 | 283 |
| | 1 injection J+1 | 12,5 | 45 |
| | Spirodiam 4 | 25 | 65 |
| | | 50 | 90 |
| | | 100 | 535 |
| | 9 injections Q1D | 1,25 | 55 |
| | | 2,5 | 60 |
| | | 5 | 110 |
| | | 10 | 160 |
| | | 20 | 300 |

* frontière de toxicité

Tableau II

| Tumeur | Protocole | Fraction de la $DL_0$ | ILS% | | | | |
|---|---|---|---|---|---|---|---|
| | | | MYKO 63 $DL_0$: 40 mg/kg | MYCLAz $DL_0$: 24 mg/kg | SOAz $DL_0$: 210 mg/kg | Spirodiam 3 $DL_0$: 75 mg/kg | Spirodiam 4 $DL_0$: 125 mg/kg |
| P388 | monoinjection à J+1 | 1/15 | NS | NS | NS | 30 | 55 |
| | | 1/5 | NS | NS | NS | 50 | - |
| | | 1/4 | 51 | 30 | 35 | - | 70 |
| | | 1/2,5 | - | - | - | - | - |
| | | 1/2 | 101 | 52 | 73 | - | 150 |
| | | 1/1,5 | - | - | - | 100 | - |
| | | 1/1,3 | - | 58 | 134 | - | - |
| | | 1 | tox. | 68 | 196 | - | - |
| P815 | monoinjection à J+1 | 1/10 | NS | NS | NS | - | 55 |
| | | 1/6 | NS | NS | NS | 55 | - |
| | | 1/5 | 25 | NS | 36 | - | 65 |
| | | 1/3 | - | NS | - | 70 | - |
| | | 1/2,5 | 49 | 27 | 71 | - | 90 |
| | | 1/1,5 | 57 | 40 | 74 | 150 | - |
| | | 1/1,25 | - | 47 | - | - | 535 |
| | | 1 | tox. | 58 | 79 | - | - |
| type de souris | | | DBA/2 femelles | CD2F1 mâles | DBA/2 femelles | CD2F1 femelles | |
| Indice thérapeutique | | P388 | 5 | 4 | 4 | ~15 | ~15 |
| | | P815 | <5 | 2,5 | 5 | > 6 | ~10 |

NS = activité non significative (ILS < 25%) tox. = toxique

**Revendications**

1. Composé de formule I:

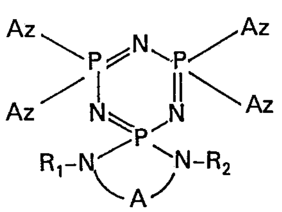

(I)

dans laquelle:

Az est un radical 1-aziridinyle non substitué ou substitué par 1 à 4 radicaux alkyles ou alkoxy en $C_1$ à $C_3$;

A est une chaîne alcoylène, alcénylène ou alcynylène, droite ou ramifiée, substituée ou non, qui peut être interrompue par un ou plusieurs radicaux divalents $-NR_3-$, -S- ou -0-, la chaîne -A- pouvant être substituée par un ou plusieurs atomes d'halogène, radicaux imino, amino, aryle non substitué ou substitué par un ou plusieurs atomes d'halogène ou radicaux alkyles en $C_1$ à $C_3$;

$R_1$, $R_2$ et $R_3$ peuvent représenter, indépendamment l'un de l'autre:
un atome d'hydrogène,
un radical $C_1$-$C_7$-alkyle,
un radical $C_1$-$C_7$-alcényle,
un radical $C_1$-$C_7$-alcynyle,
un radical monocyclique aryle,
les radicaux $R_1$, $R_2$ et $R_3$ peuvent être substitués par un atome d'halogène, les radicaux imino, amino, aryle non subsitué ou substitué par un ou plusieurs atomes d'halogène ou radicaux alkyles en $C_1$ à $C_3$.

2. Composé selon la revendication 1, caractérisé en ce que Az est un radical aziridinyle non substitué.

3. Composé selon la revendication 1 de formule I':

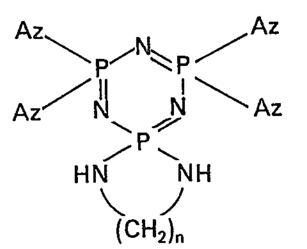

(I')

dans laquelle n est un nombre entier de 1 à 5.

4. Composé selon la revendication 3, caractérisé en ce que n = 2, 3, 4 ou 5.

5. Composé selon la revendication 4, caractérisé en ce que n = 3, ayant la formule:

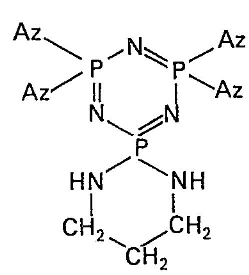

6. Composé selon la revendication 5, caractérisé en ce que n = 4, ayant la formule:

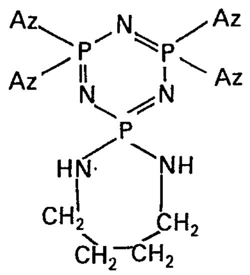

7. Composé selon la revendication 4, caractérisé en ce que n = 5.

8. Procédé de préparation d'un composé de formule I selon la revendication 1, caractérisé en ce que:

a) on fait réagir la polyamine de formule II:

$H(R_1N)—A—(NR_2)H$ (II)

sur le composé gem dichloré de formule III:

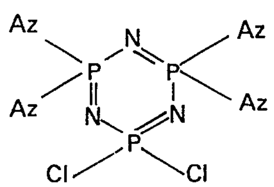

(III)

ou bien,

b) on fait réagir l'aziridine non substituée ou substituée en excès sur le composé de formule IV:

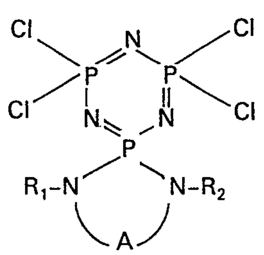

(IV)

9. Procédé selon la revendication 8, caractérisé en ce que le composé de formule III est préparé par action de l'aziridine substituée ou non sur le composé de formule V:

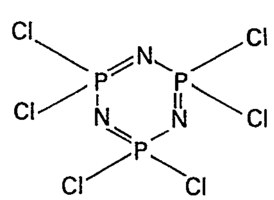

(V)

10. Procédé selon la revendication 8, caractérisé en ce que le composé de formule IV est préparé par action de la polyamine $H(R_1N—A—(NR_2)H$ sur le composé de formule V:

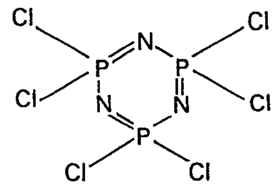

(V)

11. Procédé selon l'une des revendications 8 à 10, caractérisé en ce que la réaction est conduite dans un mélange d'éther de pétrole et de dichlorométhane.

12. Procédé selon l'une des revendications 8 à 11, caractérisé en ce que la réaction se déroule à une température comprise entre -20 °C et +20 °C.

13. Procédé selon l'une des revendications 8 à 12, caractérisé en ce que la réaction se déroule en atmosphère inerte.

14. Procédé selon l'une des revendications 8 à 13, caractérisé en ce que la polyamine est choisie parmi:

- la propylènediamine,
- la putrecine,
- la cadavérine.

15. Composition pharmaceutique contenant à titre de principe actif au moins un composé selon l'une des revendications 1 à 7.

16. Composition selon la revendication 15, caractérisée en ce qu'il s'agit d'une composition injectable.

17. Composition selon la revendication 16, caractérisée en ce qu'il s'agit d'une composition injectable par voie intra-péritonéale ou intra-veineuse.

**Patentansprüche**

1. Verbindung der Formel (I):

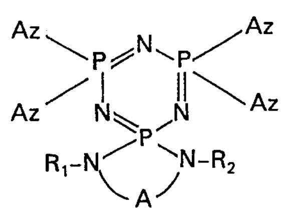

(I)

worin bedeuten:

Az einen 1-Aziridinyl-Rest, der unsubstituiert oder substituiert ist durch 1 bis 4 Alkylreste, oder einen $C_1$-$C_3$-Alkoxyrest;

A eine unverzweigte oder verzweigte, substituierte oder unsubstituierte Alkylen-, Alkenylen- oder Alkinylen-Kette, die durch einen oder mehr zweiwertige Reste $-NR_3-$, -S- oder -0- unterbrochen sein kann, wobei die Kette -A- substituiert sein kann durch ein oder mehr Halogenatome, Imino-, Amino-, Arylreste, die unsubstituiert oder substituiert sind durch ein oder mehr Halogenatome oder $C_1$-$C_3$-Alkylreste;

$R_1$, $R_2$ und $R_3$ jeweils unabhängig voneinander:

ein Wasserstoffatom,
einen $C_1$-$C_7$-Alkylrest,
einen $C_1$-$C_7$-Alkenylrest,
einen $C_1$-$C_7$-Alkinylrest,
einen monocyclischen Arylrest,
wobei die Reste $R_1$, $R_2$ und $R_3$ substituiert sein können durch ein Halogenatom, Imino-, Amino-, Arylreste, die unsubstituiert oder substituiert sind durch ein oder mehr Halogenatome oder $C_1$-$C_3$-Alkylreste.

2. Verbindung nach Anspruch 1, dadurch gekennzeichnet, dass Az für einen unsubstituierten Aziridinyl-Rest steht.

3. Verbindung nach Anspruch 1 der Formel (I'):

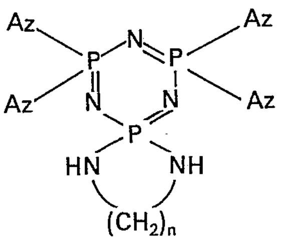

(I')

in der n eine ganze Zahl von 1 bis 5 bedeutet.

4. Verbindung nach Anspruch 3, dadurch gekennzeichnet, dass n die Zahl 2, 3, 4 oder 5 bedeutet.

5. Verbindung nach Anspruch 4, dadurch gekennzeichnet, dass n die Zahl 3 bedeutet und dass sie die Formel hat:

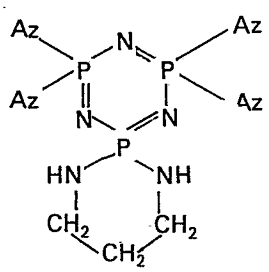

6. Verbindung nach Anspruch 5, dadurch gekennzeichnet, dass n die Zahl 4 bedeutet und dass sie die Formel hat:

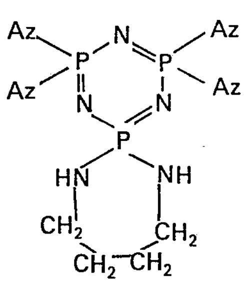

7. Verbindung nach Anspruch 4, dadurch gekennzeichnet, dass n die Zahl 5 bedeutet.

8. Verfahren zur Herstellung einer Verbindung der Formel (I) nach Anspruch 1, dadurch gekennzeichnet, dass man:

a) das Polyamin der Formel (II):

$$H(R_1N)—A—(NR_2)H \quad (II)$$

mit der gem-dichlorierten Verbindung der Formel (III) reagieren lässt:

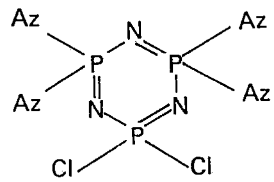

(III)

oder

b) das unsubstituierte oder substituierte Aziridin im Überschuss mit der Verbindung der Formel (IV) reagieren lässt:

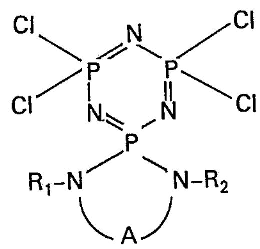

(IV)

9. Verfahren nach Anspruch 8, dadurch gekennzeichnet, dass die Verbindung der Formel (III) hergestellt wird durch Einwirkung des substituierten oder unsubstituierten Aziridins auf die Verbindung der Formel (V):

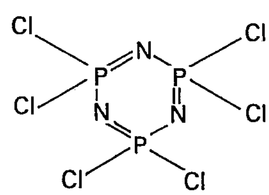

(V)

10. Verfahren nach Anspruch 8, dadurch gekennzeichnet, dass die Verbindung der Formel (IV) hergestellt wird durch Einwirkung des Polyamins $H(R_1N)—A—(NR_2)H$ auf die Verbindung der Formel (V):

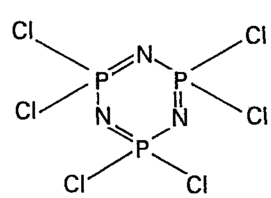

(V)

11. Verfahren nach einem der Ansprüche 8 bis 10, dadurch gekennzeichnet, dass die Reaktion in einem Gemisch aus Petroläther und Dichlormethan durchgeführt wird.

12. Verfahren nach einem der Ansprüche 8 bis 11, dadurch gekennzeichnet, dass die Reaktion bei einer Temperatur zwischen -20 °C und +20 °C abläuft.

13. Verfahren nach einem der Ansprüche 8 bis 12, dadurch gekennzeichnet, dass die Reaktion in einer inerten Atmosphäre abläuft.

14. Verfahren nach einem der Ansprüche 8 bis 13, dadurch gekennzeichnet, dass das Polyamin ausgewählt wird aus der Gruppe

- Propylendiamin,
- Putrecin,
- Cadaverin.

15. Pharmazeutische Zusammensetzung, die als Wirkstoff mindestens eine Verbindung nach einem der Ansprüche 1 bis 7 enthält.

16. Zusammensetzung nach Anspruch 15, dadurch gekennzeichnet, dass es sich dabei um eine injizierbare Zusammensetzung handelt.

17. Zusammensetzung nach Anspruch 16, dadurch gekennzeichnet, dass es sich dabei um eine auf intra-peritonealem oder intravenösem Wege injizierbare Zusammensetzung handelt.

**Claims**

1. Compound of formula I:

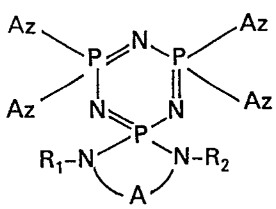

(I)

in which:

Az is a 1-aziridinyl radical not substituted or substituted by 1 to 4 alkyl or alkoxyl in $C_1$ to $C_3$ radicals;

A is an alkylene, alkenylene or alkynylene chain, straight or branched, substitued or not, which can be interrupted by one or several divalent radicals $-NR_3-$, -S- or -0-, the chain -A- able to be substituted by one or several halogen atoms, imino, amino, aryl radicals non-substituted or substituted by one or several halogen atoms or alkyl radicals in $C_1$ to $C_3$;

$R_1$, $R_2$ and $R_3$ can represent, independently of one another:

one atom of hydrogen,
one radical $C_1$-$C_7$-alkyl,
one radical $C_1$-$C_7$-alkenyl,
one radical $C_1$-$C_7$-alkynyl,
one monocyclic aryl radical,
the radicals $R_1$, $R_2$ and $R_3$ may be substituted by one atom of halogen, the radicals imino, amino, aryl nonsubstituted or substituted by one or several halogen atoms or alkyl radicals in $C_1$ to $C_3$.

2. Compound according to claim 1, characterised in that Az is a non-substituted aziridinyl radical.

3. Compound according to claim 1 of the formula I':

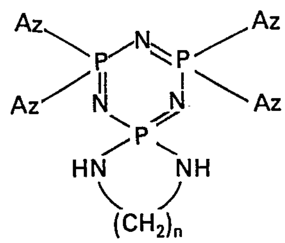

(I')

in which n is an integer from 1 to 5.

4. Compound according to claim 3, characterised in that n = 2, 3, 4 or 5.

5. Compound according to claim 4, characterised in that n = 3, having the formula:

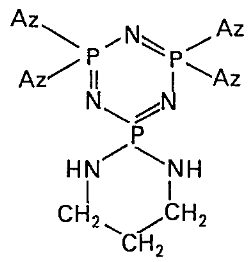

6. Compound according to claim 5, characterised in that n = 4, having the formula:

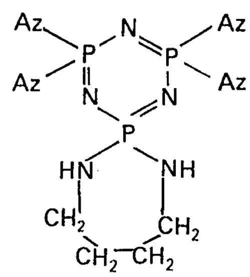

7. Compound according to claim 4, characterised in that n = 5.

8. Process for the preparation of a compound of formula I according to claim 1, characterised in that:

a) the polyamine of formula II:

$$H(R_1N)—A—(NR_2)H \quad (II)$$

is reacted with the gem-dichlorinated compound of formula III:

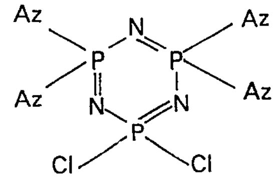

(III)

or otherwise,

b) non-substituted or substituted aziridine in excess is reacted with the compound of formula IV:

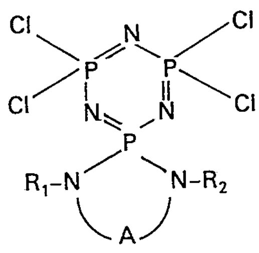

(IV)

9. Process according to claim 8, characterised in that the compound of formula III is prepared by the action of aziridine substituted or not on the compound of formula V:

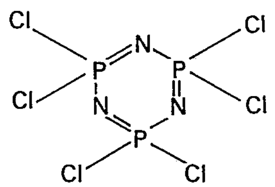

(V)

10. Process according to claim 8, characterised in that the compound of formula IV is prepared by action of the polyamine $H(R_1N)$—A—$(NR_2)H$ on the compound of formula V:

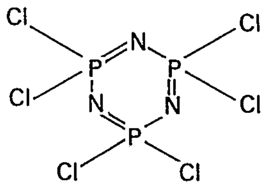

(V)

11. Process according to one of claims 8 to 10, characterised in that the reaction is carried out in a mixture of ether, petroleum and dichloromethane.

12. Process according to one of claims 8 to 11, characterised in that the reaction proceeds at a temperature between -20 °C and +20 °C.

13. Process according to one of claims 8 to 12, characterised in that the reaction proceeds in an inert atmosphere.

14. Process according to one of claims 8 to 13, characterised in that the polyamine is chosen from amongst:
- propylene diamine,
- putrescine,
- cadaverine.

15. Pharmaceutical composition containing as its active principle at least one compound according to one of claims 1 to 7.

16. Composition according to claim 15, characterised in that it is an injectable composition.

17. Composition according to claim 16, characterised in that it is an intra-peritoneal or intravenous injectable composition.

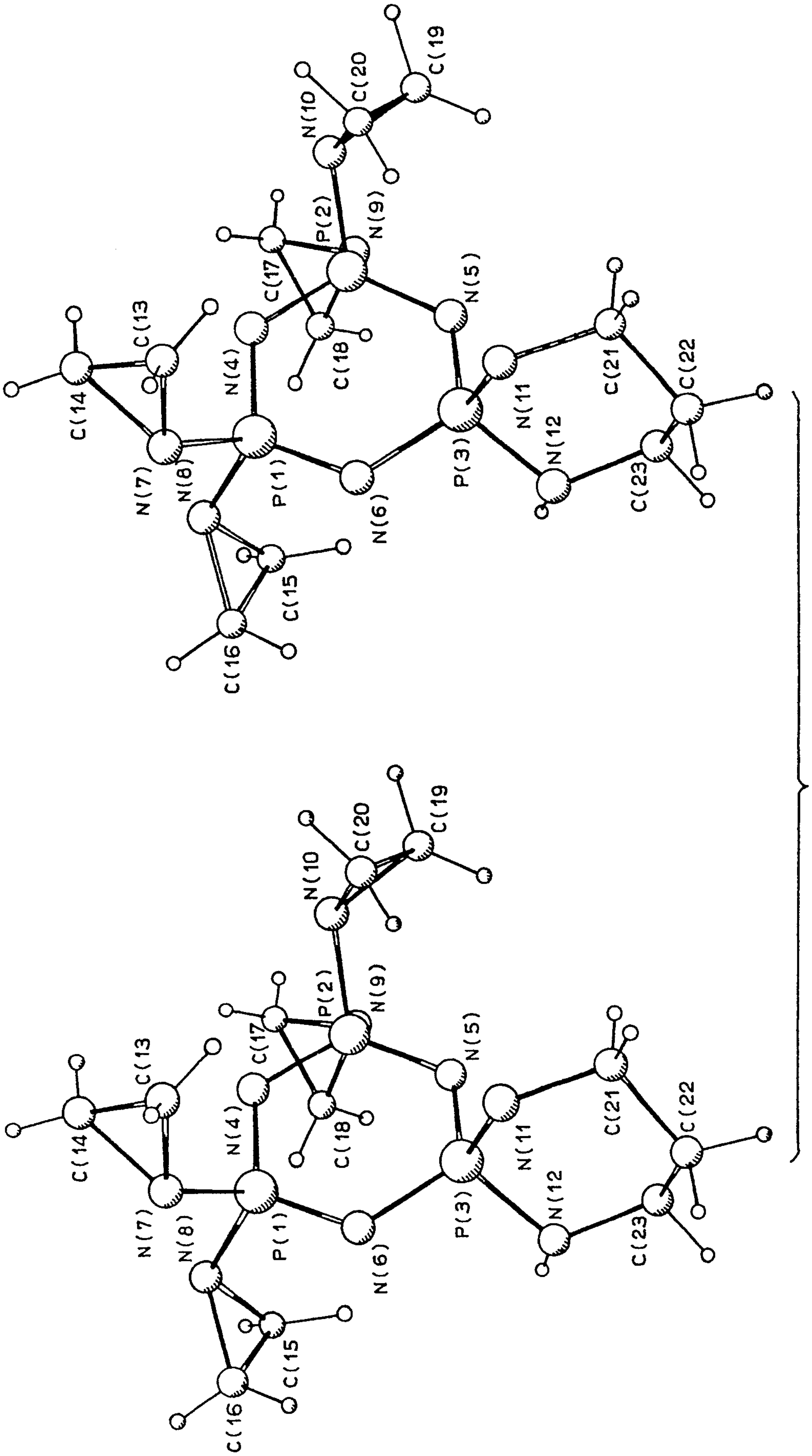

FIG_1

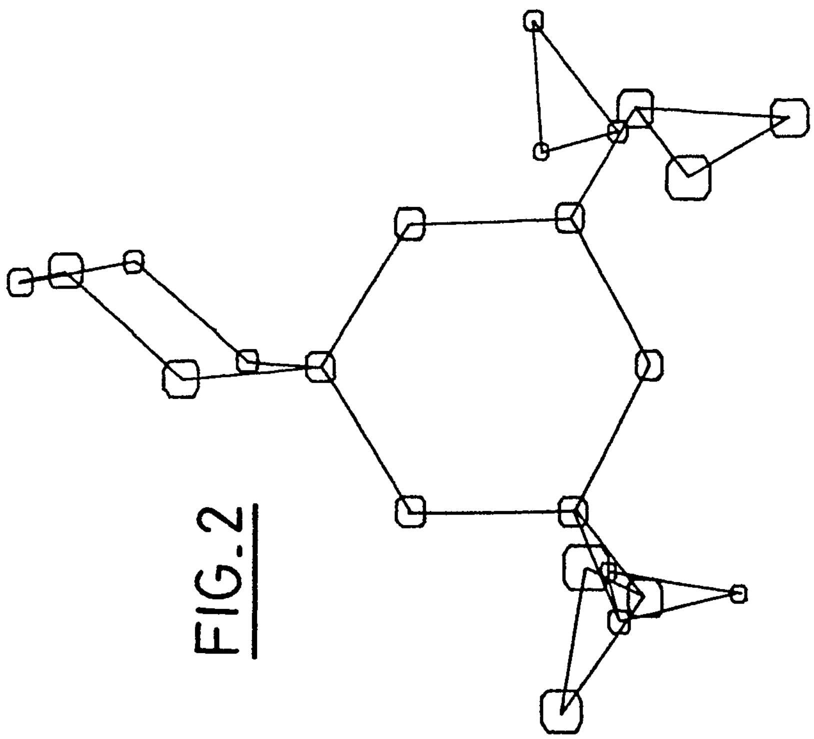

FIG. 2

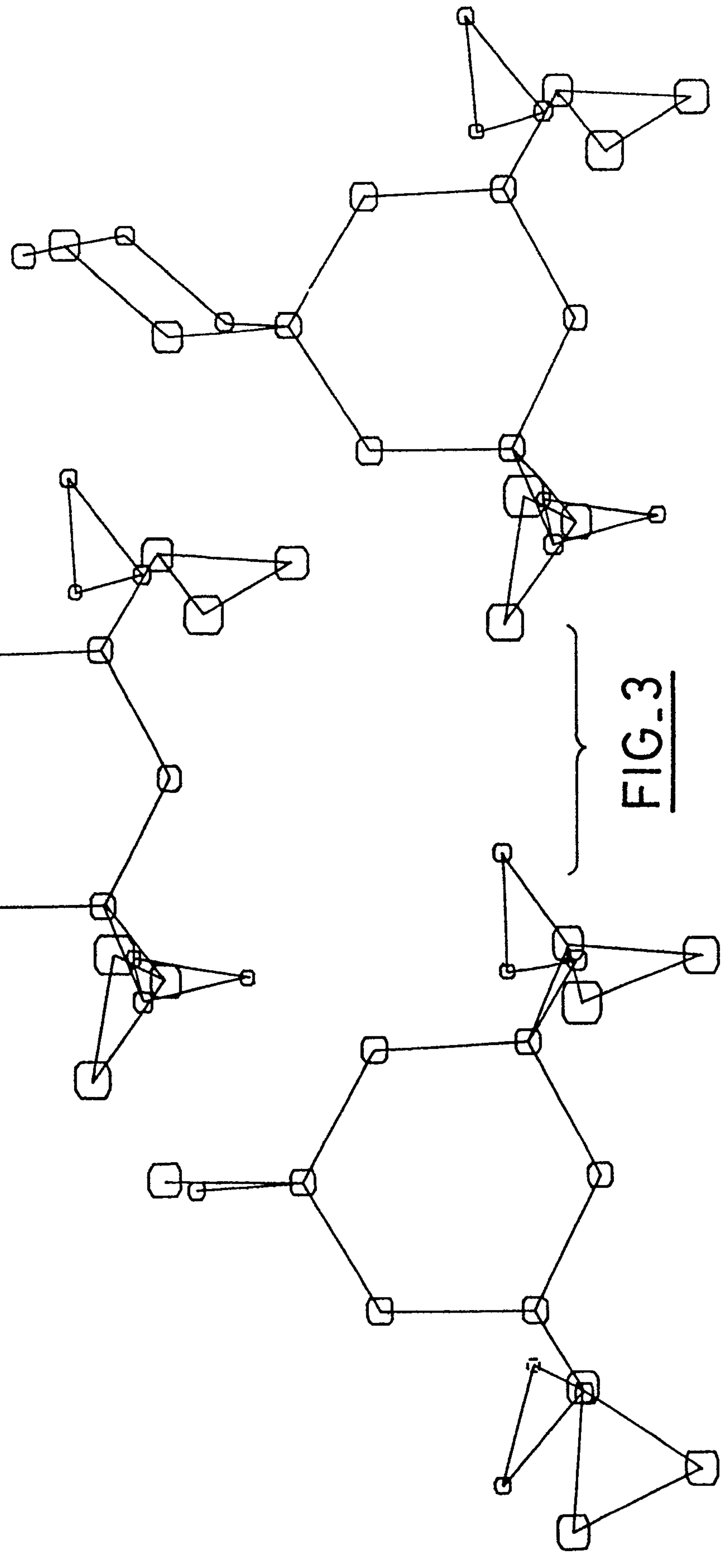

FIG. 3

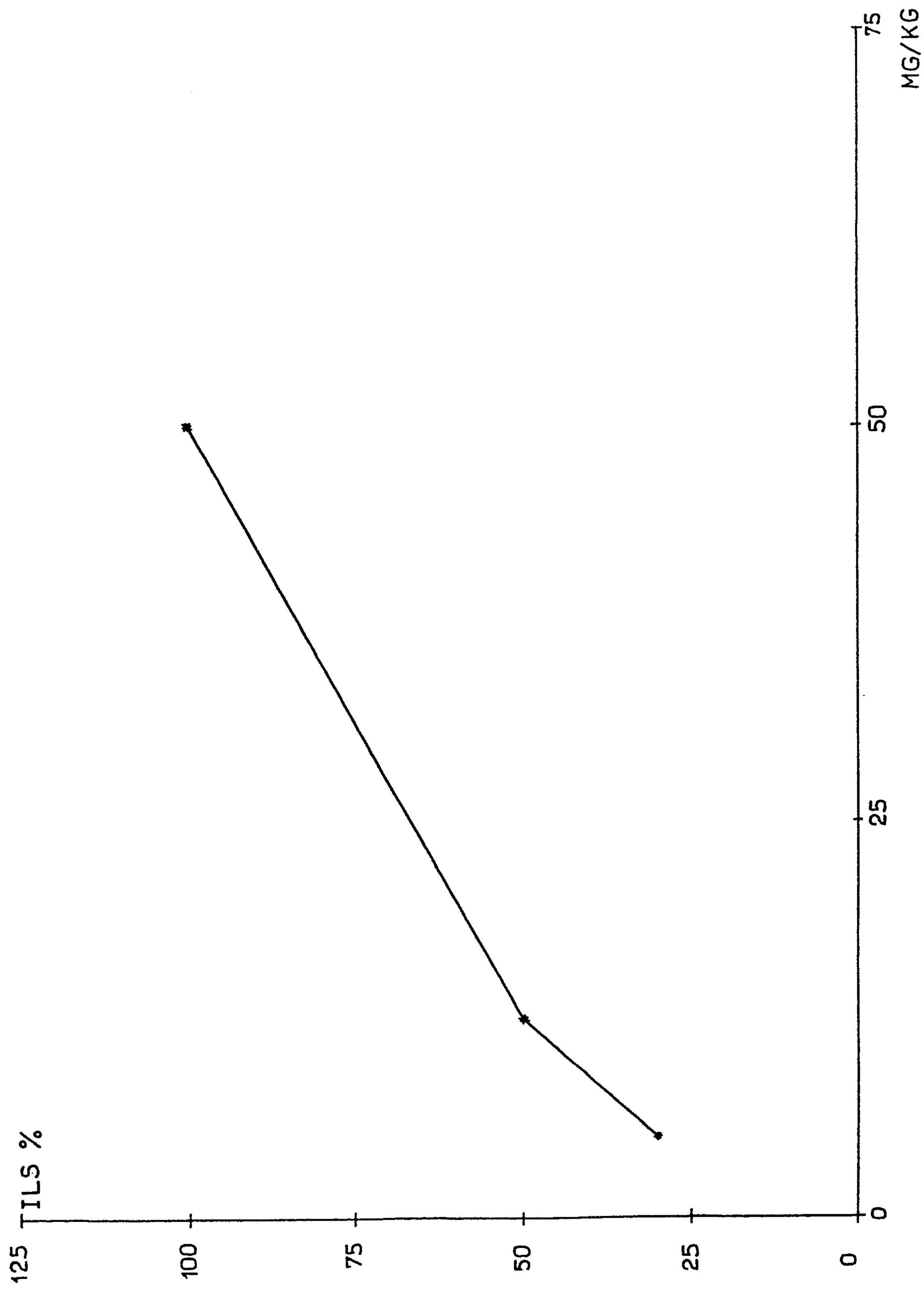


FIG. 4

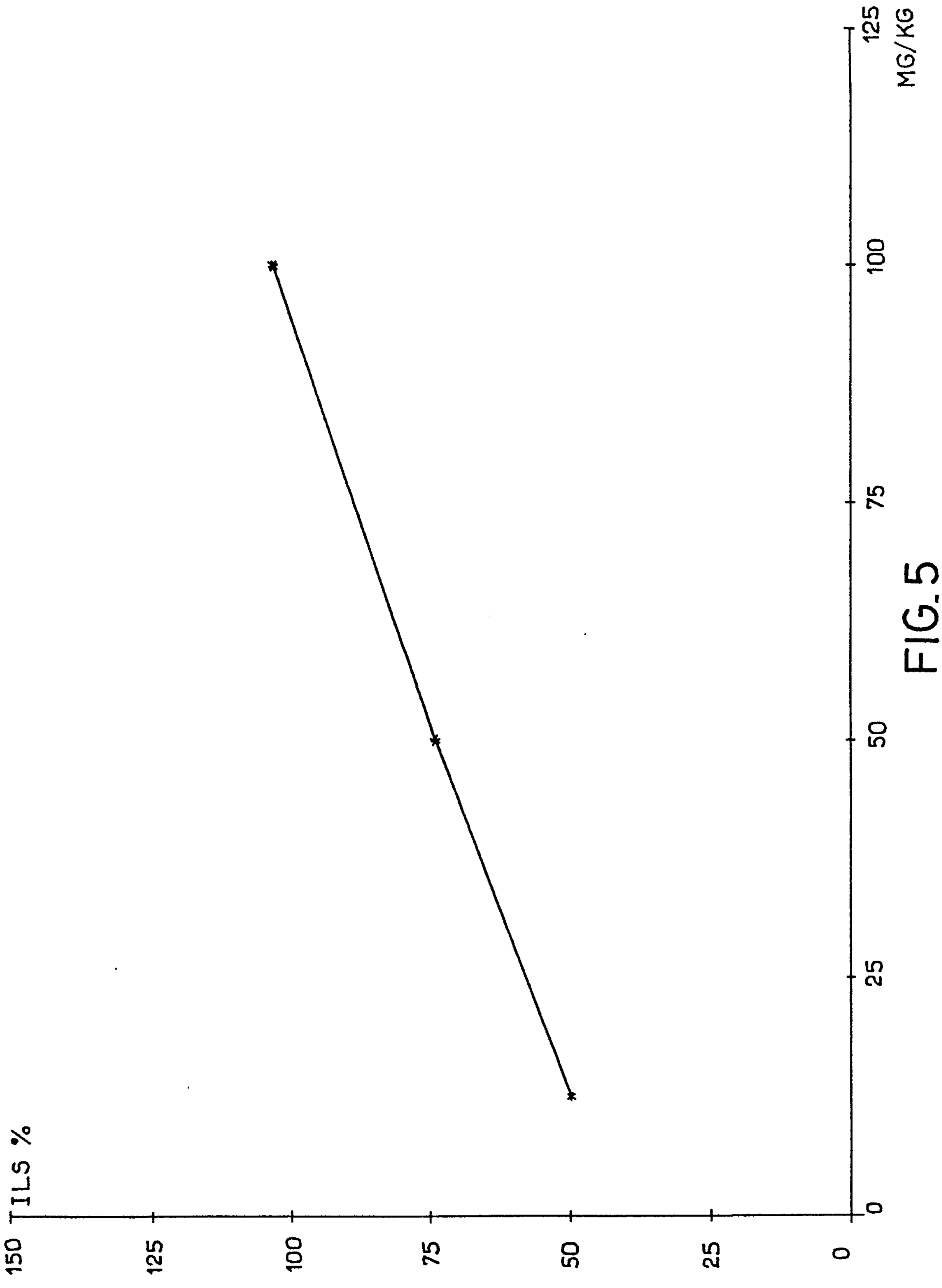


FIG.5

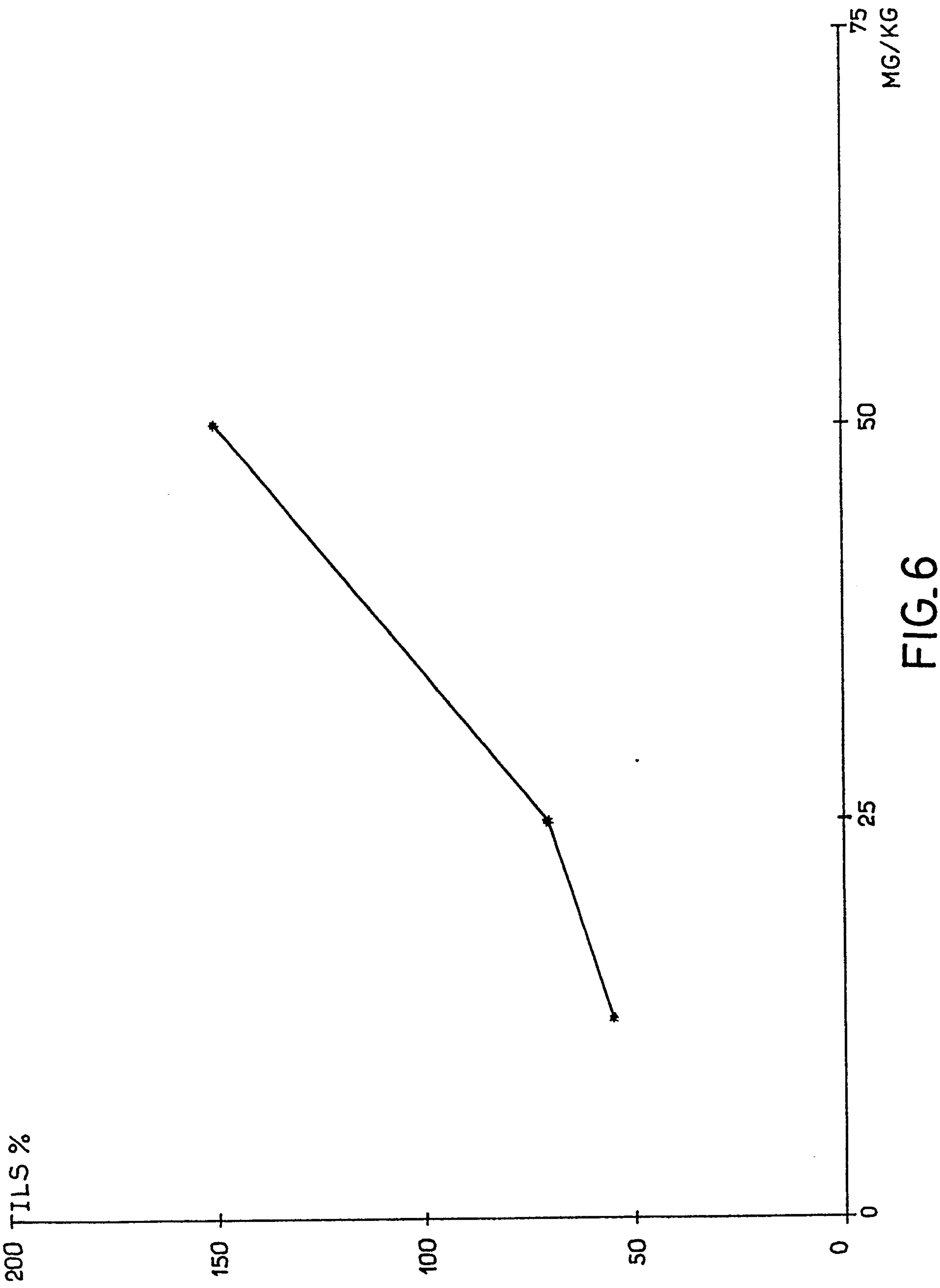


FIG.6

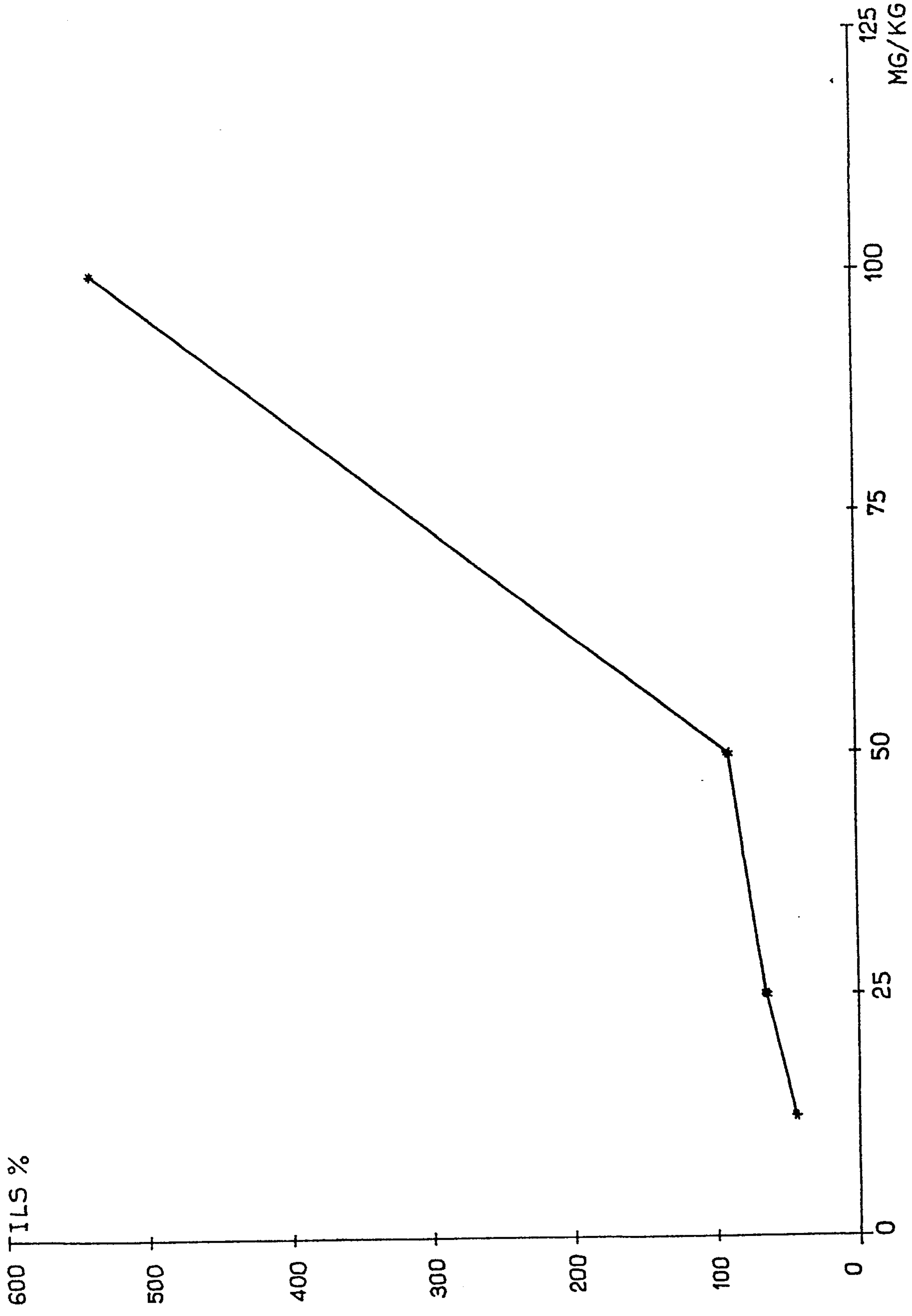


FIG_7

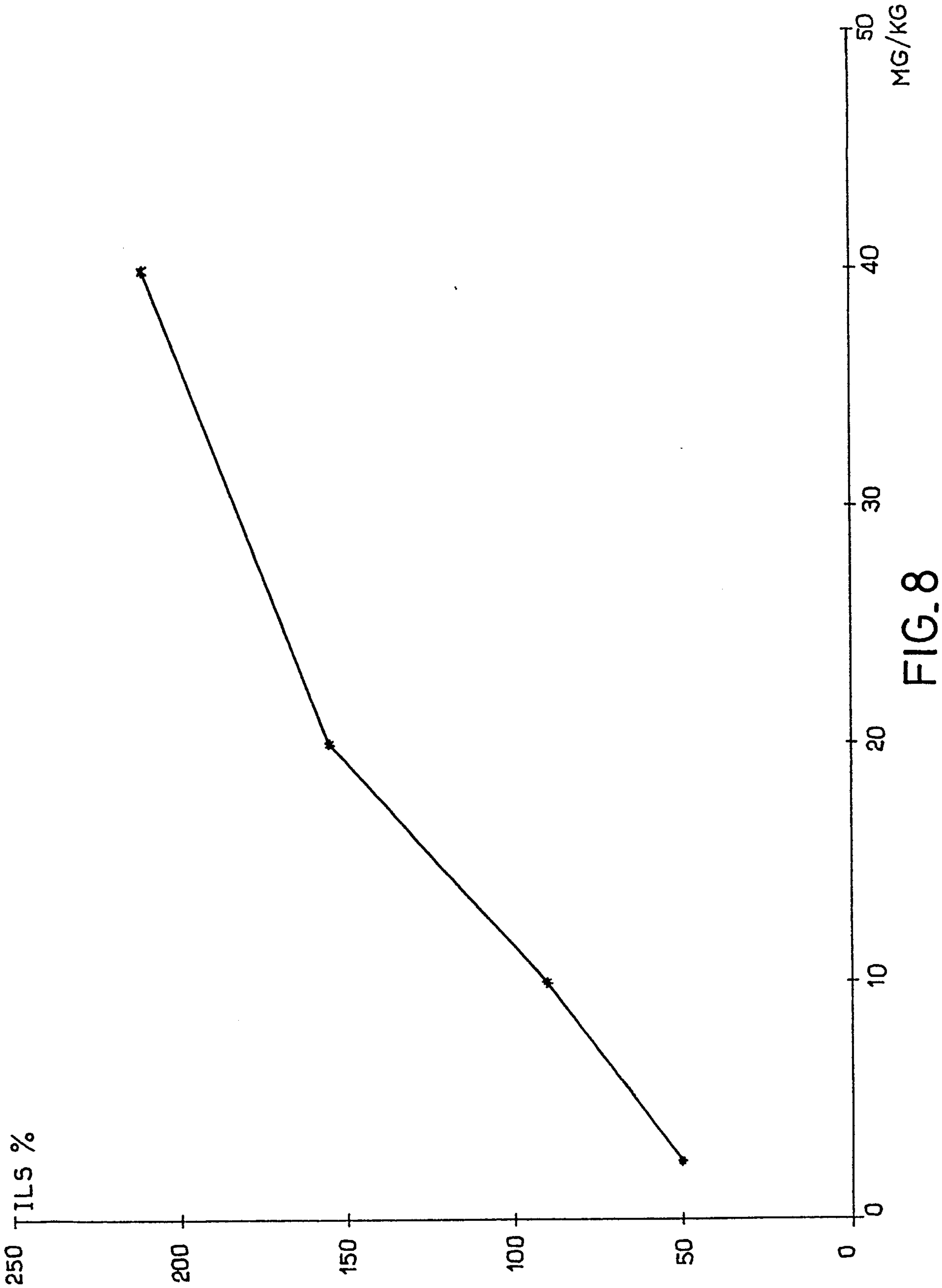


FIG. 8

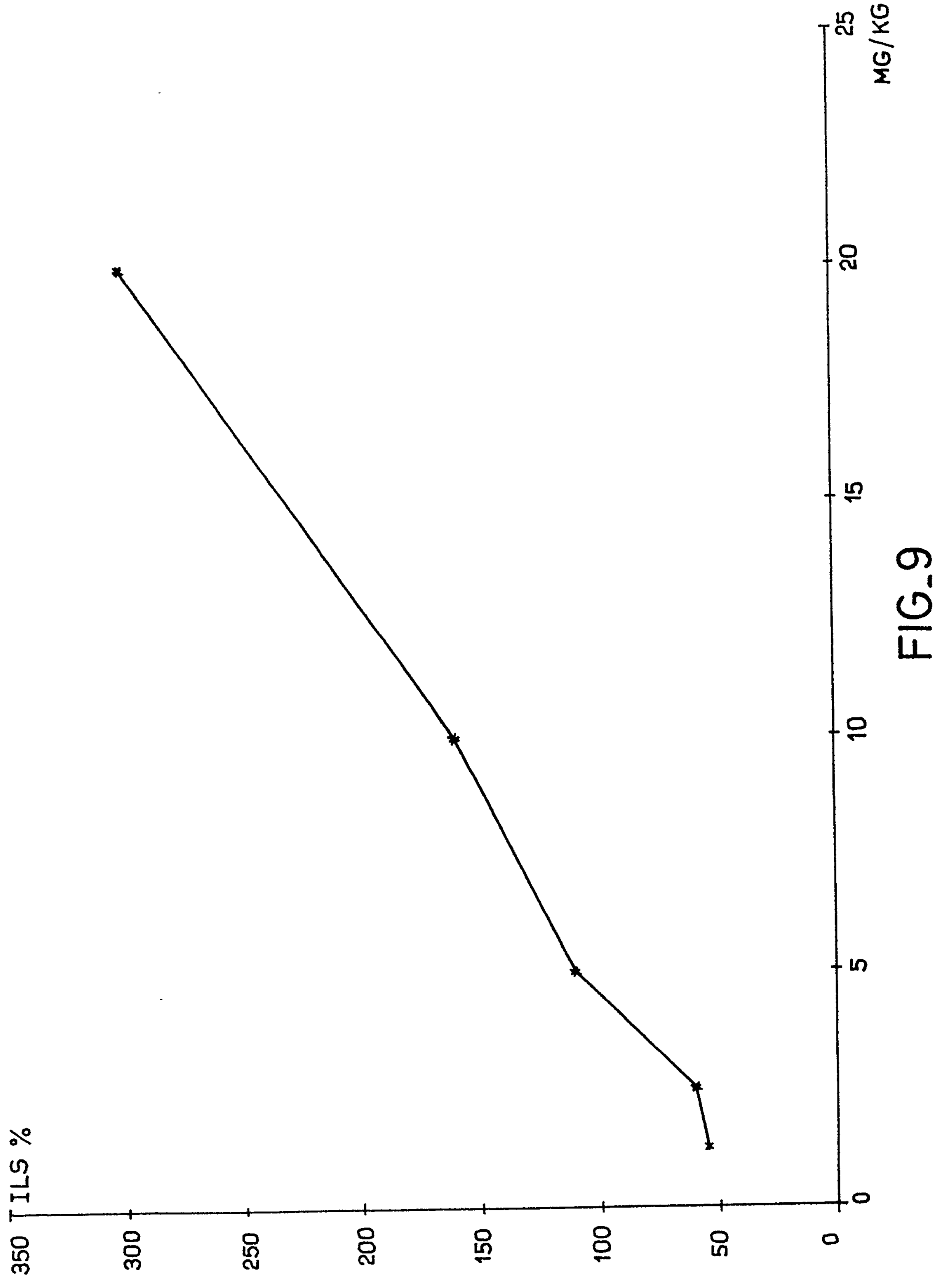


FIG_9